# EUROPEAN PATENT APPLICATION

(11) **EP 4 442 258 A1**
(43) Date of publication of application: **09.10.2024**
(21) Application number: 22900818.0
(22) Date of filing: 20.01.2022
(51) Int. Cl.: A61K 31/085, A61K 35/618, A61P 13/08, A61P 35/00, A61P 35/04, A61P 43/00

(54) **GROWTH INHIBITOR FOR METASTATIC HUMAN PROSTATE CANCER CELLS**

(30) Priority: 30.11.2021 JP 2021194178
(71) Applicant: Watanabe Oyster Laboratory Co., Ltd., Hachioji-Shi Tokyo 192-0154 (JP)
(72) Inventor: WATANABE Mitsugu, Hachioji-shi, Tokyo 192-0154 (JP)
(74) Representative: Plougmann Vingtoft a/s
(86) International application number: PCT/JP2022/001971
(87) International publication number: WO 2023/100380

(57) **Abstract**

As a result of examination of actions of DHMBA on metastatic human prostate cancer cells (PC-3 and DU-145), it has been confirmed that the DHMBA inhibits growth of the prostate cancer cells, as well as enhances cell death and exerts a growth control action on the cancer cells, and further inhibits metastatic activity of the cancer cells. Thus, an object of the present invention is for the DHMBA to provide one that would be a valuable drug means for treatment of human prostate cancer.

[Solution]

The present invention has a growth inhibitory action on metastatic human prostate cancer cells with an active ingredient of 3,5-dihydroxy-4-methoxybenzyl alcohol (DHMBA).

## Description

### TECHNICAL FIELD

The present invention relates to a growth inhibitor of metastatic human prostate cancer cells.

### BACKGROUND ART

Human prostate cancer is a metastatic cancer that is the most common cancer of males and ranks the second in cancer-related causes of death of males.

In particular, prostate cancer is known to preferentially metastasize to bone. In a variety of cancers, bone metastases are a common complication, and when comparing their incidences, they are known to exhibit 70 to 79% in multiple myeloma, 65 to 90% in prostate cancer, 65 to 75% in breast cancer, 17 to 64% in lung cancer, and 10% in colorectal cancer. Thus, it is found that bone metastatic nature of prostate cancer is extremely high. Bone metastases of prostate cancer cause complications, such as severe pain, fractures, spinal cord compression, and bone marrow inhibition. Local prostate cancer has a five-year survival rate of 100%, whereas metastatic prostate cancer has a survival rate of only 30%. Therefore, more effective therapeutic inhibitors are required. Current therapies include surgical resection, chemotherapy, hormonal castration, immunotherapy, radioisotope therapy, and the like. However, these therapeutic inhibitors are insufficient, and therefore, the development of more effective therapeutic inhibitors is expected.

### [Citation List]

### [Patent Documents]

Patent Document 1: JP-A-2017-132753

### DISCLOSURE OF THE INVENTION

### PROBLEMS TO BE SOLVED BY THE INVENTION

3,5-dihydroxy-4-methoxybenzyl alcohol (referred to herein as DHMBA), a novel phenolic antioxidant, is a novel compound isolated from marine oysters. The DHMBA is known to play roles in regulating inhibition of oxidative stress in cells. However, actions on metastatic human prostate cancer cells are completely unknown.

Therefore, the present inventors examined the actions of the DHMBA on metastatic human prostate cancer cells (PC-3 and DU-145). Consequently, the DHMBA was invented to inhibit the growth of prostate cancer cells, as well as to enhance the cell death and to exert a growth control action on cancer cells, and further to inhibit the metastatic activity of cancer cells. Thus, the DHMBA would be considered to be valuable drug means for the treatment of human prostate cancer.

### SOLUTIONS TO THE PROBLEMS

The present invention has:
a growth inhibitory action on metastatic human prostate cancer cells with an active ingredient of 3,5-dihydroxy-4-methoxybenzyl alcohol (DHMBA); or
a cell death inducing action on metastatic human prostate cancer cells with an active ingredient of 3,5-dihydroxy-4-methoxybenzyl alcohol (DHMBA); or
inhibitory actions on migration and invasion of metastatic human prostate cancer cells with an active ingredient of 3,5-dihydroxy-4-methoxybenzyl alcohol (DHMBA).

### EFFECTS OF THE INVENTION

Human prostate cancer is a metastatic cancer that ranks the top in the cancer-related causes of death of males. Accordingly, further development of more effective therapeutic inhibitors is required. In the present invention, effects of synthesized 3,5-dihydroxy-4-methoxybenzyl alcohol (DHMBA) including a novel 3,5-dihydroxy-4-methoxybenzyl alcohol (DHMBA) identified from marine oysters on human prostate cancer cells (PC-3 and DU-145) were examined.

It has been found that colony formation and cell growth of cancer cells cultured in a culture fluid containing the DHMBA (1 and 10 µM) are predominantly inhibited.

This action had no significant inhibitory effect in the presence of inhibitors of signaling factors involved in intracellular signaling.

This suggested that the DHMBA acted on the intracellular signaling system. Furthermore, as the mechanism, when expression levels of Ras, PI3K, Akt, MAPK, and mTOR of molecules involved in the signaling system that enhance the cell growth were examined, they reduced.

Interestingly, the expression levels of p53, p21, Rb, and regucalcin of a cancer cell inhibition gene protein were increased.

On the other hand, the DHMBA promoted cell death. The DHMBA, an antioxidant, inhibited production of active oxygen in cancer cells. It was suggested that a cancer cell growth inhibitory action of the DHMBA was associated with gene expression via an aryl hydrocarbon receptor.

In addition, the expression levels of NF-kB, caveolin-1, and integrin β1, which were protein factors involved in the metastasis of cancer cells, were decreased.

Thus, the novel natural compound, the DHMBA, was confirmed to inhibit the growth of prostate cancer cells and also enhance their cell deaths, to exert a growth control action on cancer cells, and further to inhibit the metastatic activity of cancer cells.

Thus, the DHMBA would be considered as valuable means for the treatment of human prostate cancer.

### BRIEF DESCRIPTION OF THE DRAWINGS

Fig. 1 is an explanatory diagram describing an inhibitory effect of 3,5-dihydroxy-4-methoxybenzyl alcohol on growth of human prostate cancer PC-3 cells.
Fig. 2 is an explanatory diagram describing an inhibitory effect of 3,5-dihydroxy-4-methoxybenzyl alcohol (DHMBA) on growth of human prostate cancer DU-145 cells.
Fig. 3 is an explanatory diagram describing an inhibitory effect of 3,5-dihydroxy-4-methoxybenzyl alcohol (DHMBA) on growth of human prostate cancer PC-3 cells in the presence of a cell growth inhibitor and an intracellular signaling inhibitor.
Fig. 4 is an explanatory diagram describing an effect of the DHMBA on an expression level of protein molecules involved in enhancement of cell growth of prostate cancer cells.
Fig. 5 is an explanatory diagram describing an effect of 3,5-dihydroxy-4-methoxybenzyl alcohol (DHMBA) on death of human prostate cancer PC-3 and DU-145 cells.
Fig. 6 is an explanatory diagram describing the effects of 3,5-dihydroxy-4-methoxybenzyl alcohol (DHMBA) on the death of human prostate cancer PC-3 cells and the comparison with an anticancer drug, gemcitabine.
Fig. 7 is an explanatory diagram describing effects of an aryl hydrocarbon receptor inhibitor on growth inhibitory and cell death inducing actions of the DHMBA on prostate cancer cells.
Fig. 8 is an explanatory diagram describing an inhibitory effect of the DHMBA on active oxygen production of prostate cancer cells.
Fig. 9 is an explanatory diagram describing an inhibitory effect of the DHMBA on migration of prostate cancer cells.
Fig. 10 is an explanatory diagram describing an inhibitory effect of the DHMBA on invasion of prostate cancer cells.
Fig. 11 is an explanatory diagram describing an effect of the DHMBA on an expression level of protein molecules involved in enhancement of migration and invasion of prostate cancer cells.

### DESCRIPTION OF PREFERRED EMBODIMENTS

In the present invention, experiments were conducted to find whether 3,5-dihydroxy-4-methoxybenzyl alcohol (DHMBA) would be an active ingredient, such as an inhibitor of metastatic human prostate cancer.

### (Experimental Materials and Methods)

### (Reagents)

A culture fluid containing a Dulbecco's Modified Eagle Medium (DMEM) culture fluid (4.5 g/glucose, L-glutamine-pyruvate, and an antibiotic drug (100 units/mL of penicillin, 100 µg/mL of streptomycin, and 1% of P/S) was obtained from Corning (Mediatech, Inc. Manassas, VA, USA). Fetal bovine serum (FBS) was purchased from HyClone (Logan, UT, USA). Amphotericin B (fungizone), a caspase-3 inhibitor (CAS 169332-60-9-Calbiochem), gemcitabine, and 2-Methyl-2H-pyrazole-3-carboxylic acid (2-methyl-4-o-tolylazo-phenyl)-amide (CH223191) were purchased from Selleckchem.com (Houston, TX, USA). All other reagents were purchased from Sigma-Aldrich (USA). The caspase-3 inhibitor was dissolved in phosphate-buffered saline (PBS) and the CH223191 was also dissolved in dimethyl sulfoxide (DMSO). The DHMBA and the other reagents were dissolved in 100% of ethanol and stored cold at -20°C until used for the experiment.

### (3,5-Dihydroxy-4-Methoxybenzyl Alcohol (DHMBA))

As 3,5-dihydroxy-4-methoxybenzyl alcohol (DHMBA), a synthetic compound with purity of 100% was used. It was dissolved in 100% of ethanol and stored cold at -20°C until used for the experiment. Note that it has been proven from the patent obtained by the present inventors that a large amount of 3,5-dihydroxy-4-methoxybenzyl alcohol (DHMBA) is contained in an oyster meat extract extracted from oyster meat, and it is convinced that the use of such 3,5-dihydroxy-4-methoxybenzyl alcohol (DHMBA) produces the same result.

### (Human Prostate Cancer Cells)

The metastatic human prostate cancer cells, PC-3 and DU-145 cells, were purchased from American Type Culture Collection (ATCC CRL-1435^{™}, ATCC, Rockville, MD, USA). The PC-3 cells were isolated from a cancer metastasized to bone by adenocarcinoma of a 62-year-old adult male. The DU-145 cells were isolated from a cancer metastasized to a brain by adenocarcinoma of a 69-year-old adult male. These cancer cells were cultured in a Dulbecco's Modified Eagle Medium culture fluid (4.5 g/glucose, L-glutamine-pyruvate, and an antibiotic drug (containing 100 units/mL of penicillin, 100 µg/mL of streptomycin, 1% of P/S, and 1% of fungizone).

### (Forming Colony)

The PC-3 and DU-145 cells (1 × 10³/in 2 ml of a culture fluid per hole using a six-hole plate) were cultured in a DMEM culture fluid (containing 10% of FBS, 1% of P/S, and 1% of fungizone) containing the DHMBA (1 and 10 µM) under 5% of CO₂, at 37°C, for nine days. After the culture, the culture fluid was removed, the cells were washed twice with PBS, 95% of methanol (0.5 ml) was added, and the cells were fixed for 20 minutes. After the fixation, each well of a culture dish was washed four times with PBS (1 ml), and after that 0.5% of crystal violet (0.5 ml) was added, and the product was left for two hours at room temperature for staining. After the stain solution was washed to be removed, drying was performed, and after that colonies containing 50 or more cells were counted by a microscope (Olympus MTV-3, Olympus Corporation, Tokyo, Japan).

### (Cell Growth)

The PC-3 and DU-145 cells (the cell number of 1 × 10⁵/in 1 ml of a culture fluid per hole using a 24-hole plate) were cultured in a DMEM culture fluid (containing 10% of FBS, 1% of P/S, and 1% of fungizone) containing the DHMBA (1 and 10 µM) under 5% of CO₂, at 37°C, for one, two, three, four, and six days. In another experiment, the PC-3 cells (the cell number of 1 × 10⁵/ml per well) were cultured in a DMEM culture fluid (containing 10% of FBS, 1% of P/S, and 1% of fungizone) for three days in the presence of the DHMBA (10 µM), cell growth cycle inhibitors roscovitine (10 or 100 nM), butyrate (10 or 100 µM), and sulforaphane (1 or 10 nM) or intracellular signaling inhibitors wortmannin (10 or 100 nM), PD98059 (1 or 10 µM), and staurosporine (1 or 10 nM), and further an aryl hydrocarbon receptor (AHR) inhibitor CH223191 (1, 10, or 25 µM). After the culture, 0.1 ml of 0.05% trypsin-EDTA solution obtained by dissolving the cells adhering to the bottom surface of the culture dish in PBS (Thermo Fisher Scientific, Waltham, MA, USA) not containing Ca²⁺/Mg²⁺ was added, incubation was performed at 37°C for two minutes to release the adhering cells, and 0.9 ml of the DMEM culture fluid containing 10% of FBS and 1% of P/S was added to stop the reaction. The cell number in the cell suspension was counted by the method described in the item of "Measurement of Cell Number."

### (Cell Death)

The PC-3 and DU-145 cells (1 × 10⁵/in 1 ml of a culture fluid per hole using a 24-hole plate) were cultured in a DMEM culture fluid (containing 10% of FBS, 1% of P/S, and 1% of fungizone) under 5% of CO₂, at 37°C, for three days, and when the cells reached sub-confluence, the culture fluid was exchanged for the one containing the DHMBA (0.01, 0.1, 1, 10, 100, or 1000 µM), and the cells were cultured for 24 and 48 hours. After the culture, the cells adhering to the bottom surface of the dish were removed and counted. In another experiment, after the culture for three days, when the cells reached sub-confluence, the culture fluid was exchanged for a culture fluid containing the DHMBA (1 or 10 µM) and a caspase-3 inhibitor (10 µM), and the cells were counted after the culture for 48 hours. Further, after the culture for three days, when the cells reached sub-confluence, the culture fluid was exchanged for a culture fluid obtained by adding gemcitabine (0.1, 1, 10, 50 or 100 nM) or CH223191 (1, 10, and 25 µM) to a DMEM culture fluid containing the DHMBA (1 or 10 µM) or not containing the DHMBA, and the cells were counted after the culture for 48 hours.

### (Counting Cells)

After the culture of the cells, 0.1 ml of 0.05% trypsin-EDTA solution obtained by dissolving the cells adhering to the bottom surface of the culture dish in PBS (Thermo Fisher Scientific, Waltham, MA, USA) not containing Ca²⁺/Mg²⁺ was added, incubation was performed at 37°C for two minutes to release the adhering cells, and 0.9 ml of the DMEM culture fluid containing 10% of FBS and 1% of P/S was added to stop the reaction. The visible cells were counted by the Olympus MTV-3 microscope using a hemocytometer (Sigma-Aldrich).

### (Measuring Intracellular Active Oxygen)

The intracellular active oxygen (reactive oxygen species: ROS) was measured using an ROS measurement kit (MAK144-1 Kit, Sigma-Aldrich) using pro-fluorescent substrate H₂DCFDA. The PC-3 and DU-145 cells (the cell number of 2.5 × 10⁴/per well, in 0.1 ml of a culture fluid per hole using a 96-hole plate) were cultured in a DMEM culture fluid (containing 10% of FBS, 1% of P/S, and 1% of fungizone) containing the DHMBA (0.1, 1, 10, 100, or 1000 µM) under 5% of CO₂, at 37°C, for 24 hours. After the culture, the cells were washed with PBS, 0.1 ml and 25 µM of an H₂DCFDA solution was added, and incubation was performed for 45 minutes. Afterwards, the cells were washed with PBS, and as the amount of active oxygen produced in the cells, fluorescence intensity was measured using a fluorescence measurement plate reader at a wavelength Ex/Em = 485/535 nm. The result was displayed as % of fluorescence intensity relative to the target group to which the DHMBA was not added.

### (Measuring Cell Migration)

The migration of the prostate cancer PC-3 and DU-145 cells was examined using a scratch assay. The PC-3 and DU-145 cells (the cell number of 2 × 10⁵/in 1 ml of a culture fluid per hole using a 24-hole plate) were cultured in a DMEM culture fluid containing 10% of FBS, 1% of P/S, and 1% of fungizone under 5% of CO₂, at 37°C, for 48 hours, and a straight line scratch was performed using a pipette tip (200 µl) when the cells reached confluence. Free cells were washed with PBS to be removed, the above-described culture fluid (containing 0.1, 1, 10, 100, or 1000 µM of the DHMBA) was added, and the cells were cultured for 48 hours. After the culture, intervals of cell-free parts were measured. The intervals after the culture were compared with the intervals when the scratch was performed before the culture and displayed as % with respect to the control (a value when the DHMBA was not contained).

### (Measuring Cell Invasion Activity)

The cell invasion activity of the prostate cancer PC-3 and DU-145 cells was measured using Transwell chambers (the catalogue number CLS3464-48EA of Corning, Life Sciences, USA). A chamber was placed in each well of the 24-hole plate. 50 µl of Matrigel (Corning Life Sciences, Cat. No. 356230, USA) was added to the upper surface of the chambers. After drying the gel, 500 µl of a suspension of the PC-3 or DU-145 cells (the cell number of 2.5 × 10⁴/ml, the DMEM not containing FBS) was added to the upper surface of the gel. This cell suspension contained the DHMBA (0, 1, 10, or 100 µM). After the addition of the cell suspension to the chambers, it was incubated for one hour to cause the cells to adhere. 0.75 ml of the culture fluid not containing the FBS and a culture fluid containing 10% of FBS were added to the lower surface of the chambers. The cells on the plate were cultured under 5% of CO₂, 95% of air, at 37°C, for 24 hours. After the culture, the culture fluid on the upper surface of the chambers was removed, the filter was collected and placed in 70% of cold ethanol (1 ml) for 30 minutes to fix the cells infiltrated with the gel present on the lower surface of the filter. After that, the product was immersed in a solution (containing 0.5% of crystal violet dissolved in 0.75 ml of 20% methanol) for 30 minutes for staining. After the staining, the product was washed with PBS and dried. After the drying, the number of the stained cells was counted by the microscope (Olympus MTV-3, Olympus Corporation, Tokyo, Japan). Videos of the stained cells were photographed with the microscope.

### (Western Blot)

The PC-3 cells (1 × 10⁶ cells/10 ml, 100-mm dish) were cultured in a DMEM culture fluid containing 10% of FBS, 1% of P/S, and 1% of fungizone under 5% of CO₂, at 37°C, for three days. In addition, the culture fluid contained the DHMBA (10 µM). After the culture, the cells were adjusted by adding a cell lysis buffer (Cell Signaling Technology, Danvers, MA, USA) containing proteolytic enzyme and a protein phosphatase activity inhibitor. The cell solution was centrifuged at 17,000 xg at 4°C for 10 minutes to collect the supernatant. An amount of protein in the supernatant was measured using Bio-Rad Protein Assay Dye (Bio-Rad Laboratories, Inc., Hercules, CA, USA) with albumin as reference protein. Protein samples were stored in a freezer at -80°C until being used. Using 40 µg of a protein sample solution, the product was added to SDS polyacrylamide gel electrophoresis (SDS-PAGE, 12%) and separated. After that, the protein molecules were transferred to a nylon membrane. Immunoblot was performed on the membrane using an antibody that specifically bound to the protein molecules. The antibodies, such as Ras (cat. no. 3339, rabbit), Akt (cat. no. 9272, rabbit), mitogen-activated protein kinase (MAPK; cat. no. 4695, rabbit), phosphorylated-MAPK (cat. no. 4370, rabbit), mechanistic target of rapamycin (mTOR, cat. no. 4517, mouse), Rb (cat. no. 9309, mouse), p21 (cat. no. 2947, rabbit), caveolin-1 (cat. no. 3267, rabbit), and β-actin (cat. no. 3700, mouse), were purchased from Cell Signaling Technology (Danvers, MA, USA). Also, p53 (cat. no. sc-126, mouse), NF-κB p65 (cat. no. sc-109, rabbit), and integrin β1 (cat. no. sc-13590, mouse) were obtained from Santa Cruz Biotechnology, Inc. (Santa Cruz, CA, USA). In addition, rabbit anti-regucalcin antibody was purchased from Sigma-Aldrich (cat. no. HPA029103, rabbit). Target proteins were incubated at low temperature for 16 hours using the primary antibodies described above. After that, using a secondary antibody of horseradish peroxidase-conjugated secondary antibodies (Santa Cruz Biotechnology, Inc., mouse sc-2005 or rabbit sc-2305; diluted 1: 1,000), incubation was performed for 60 minutes at room temperature. After that, protein bands were illuminated with chemiluminescence substrate (cat. no. 34577, Thermo Scientific, Rockford, IL, USA) and exposed and detected on X-ray films using a developer.

### (Statistical Processing)

The statistical processing of data was performed using GraphPad InStat version 3 for Windows XP (GraphPad Software Inc., La Jolla, CA, USA). The data were displayed as mean values ± standard deviations. Multiple comparison tests were performed with the Tukey-Kramer multiple comparison test. A significance level of 0.05% or less was determined significant.

### (Experimental Results)

### (Effect of DHMBA on Growth of Human Prostate Cancer Cells)

First, the effect of the DHMBA on colony formation of the metastatic human prostate cancer PC-3 and DU-145 cells was examined.

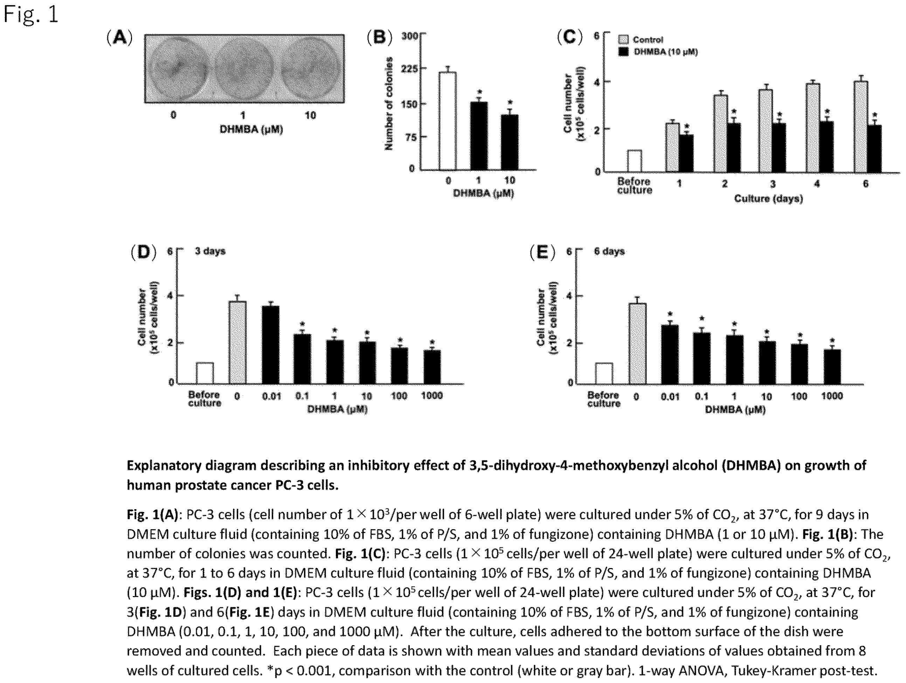

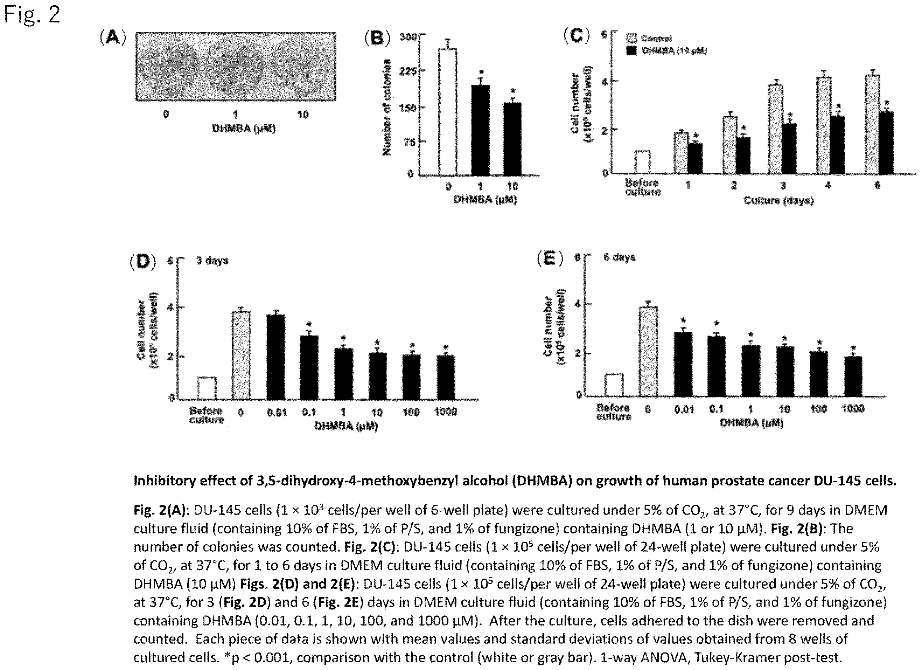

The colony formation of the PC-3 and DU-145 cells was significantly inhibited when the cells were cultured in the presence of the DHMBA (1 or 10 µM) for nine days (see Fig. 1 and Fig. 2).

Further, when the effect of the DHMBA on the cell growth was examined, the growth of PC-3 cells and DU-145 cells was inhibited by culturing them in the presence of the DHMBA (10 µM) for one to six days. The significant effect of the DHMBA on cell growth was also recognized at a low concentration of 0.01 µM (see Fig. 1 and Fig. 2).

Next, to know the mechanism of action of the effect of the DHMBA, the effect of the coexistence of various inhibitors in the presence of the DHMBA was examined.

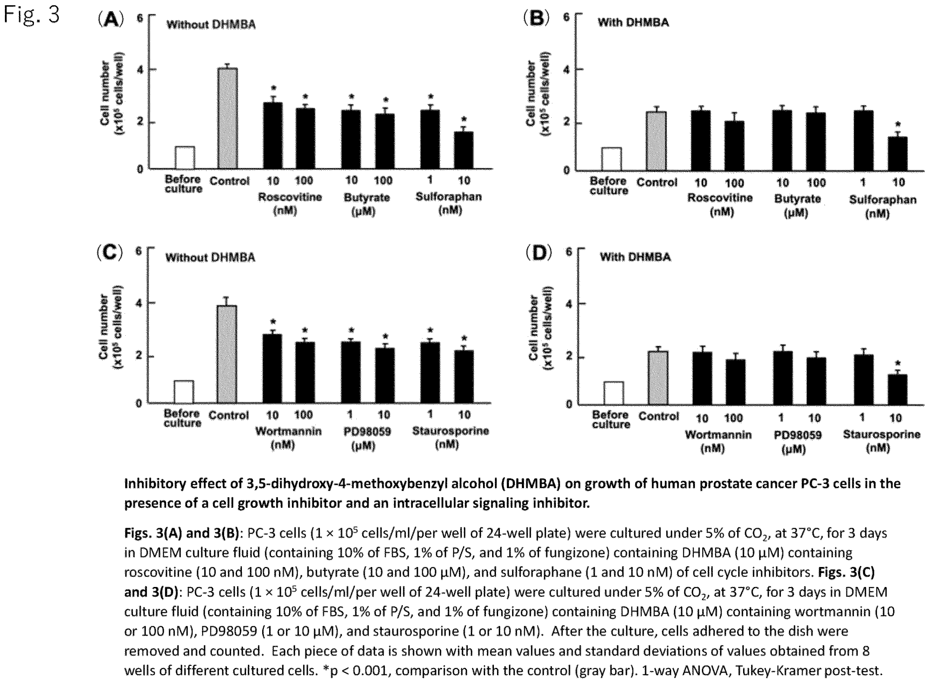

The cell growth was inhibited when the PC-3 and DU-145 cells were cultured for three days in the presence of cell cycle inhibitors, roscovitine (10 or 100 nM), butyrate (10 or 100 µM), and sulforaphane (1 or 10 nM) (Fig. 3A).

The cell growth inhibitory effect of the DHMBA (10 µM) was not observed in the presence of these inhibitors (Fig. 3B).

These results suggested that the DHMBA acted in the G1 and G1/M phases of cell growth cycle.

Furthermore, whether the cell growth inhibitory effect of the DHMBA on prostate cancer cells was based on the action to the signaling process that led to the growth in the cells was examined using these inhibitors.

The cell growth was inhibited when the prostate cancer cells (PC-3) were cultured in the presence of wortmannin (10 or 100 nM), PD98059 (1 or 10 µM), and staurosporine (1 or 10 nM) for three days (Fig. 3C). In the presence of these inhibitors, no significant cell growth inhibitory effect of the DHMBA (10 µM) was expressed (Fig. 3D). These results suggested that the expression of the cell growth inhibitory action of the DHMBA acted on the signaling process promoting the cell growth.

### (Effect of DHMBA on Expression Level of Protein Related to Cell Growth)

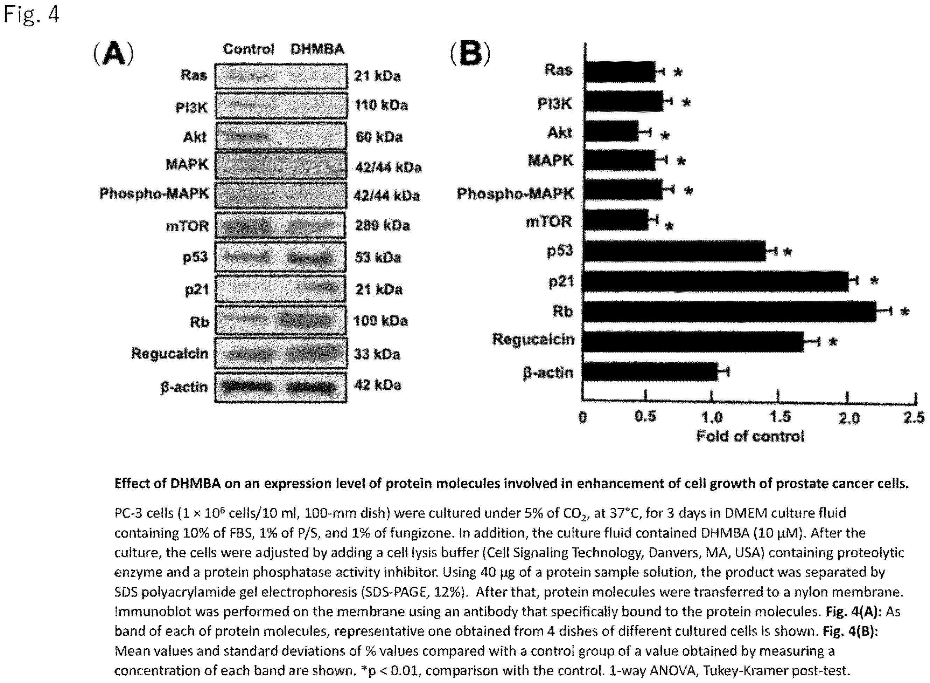

To clarify the molecular mechanism of the action by which the DHMBA inhibits the growth of the prostate cancer cells (PC-3), variation in the expression levels of protein molecules involved in the enhancement of cell growth was examined by western blotting (Fig. 4).

The PC-3 cells were cultured in the presence of the DHMBA (10 µM) for three days. The expression levels of Ras, PI3 kinase, Akt, MAP kinase, phospho-MAP kinase, and mTOR, which contributed to the enhancement of cell growth, were significantly inhibited. Interestingly, the expression levels of p53, Rb, p21, and regucalcin of the molecules inhibiting the cell growth were significantly increased. It was suggested that the variations in these protein molecules contributed to the expression of the cell growth inhibitory effect of the DHMBA.

### (Effect of DHMBA on Cell Death Induction)

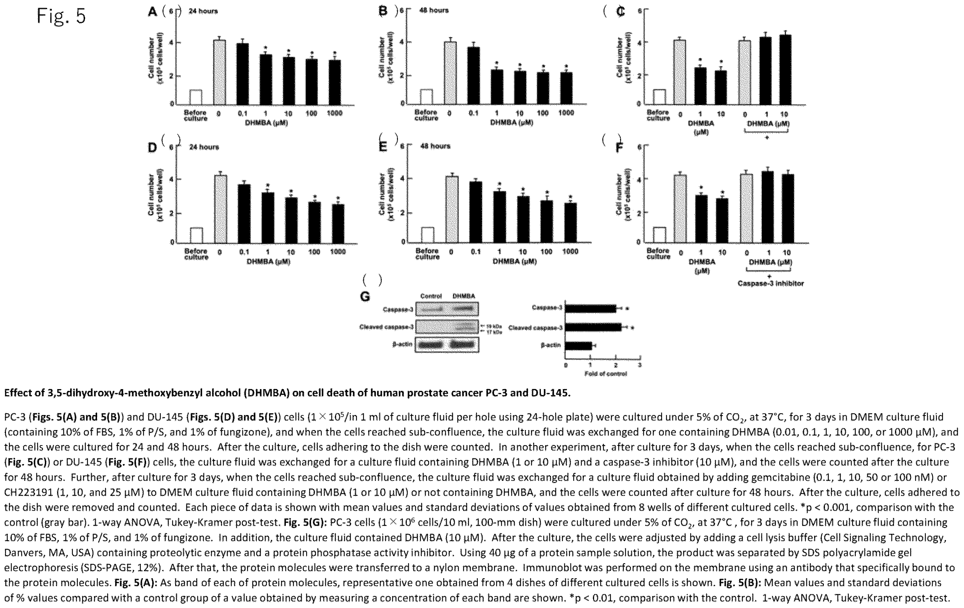

In addition, the cell death inducing action of the DHMBA on the prostate cancer cells was examined. The PC-3 and DU-145 cells were cultured in the absence of the DHMBA for three days, and when reaching sub-confluency, the culture fluid was exchanged for a culture fluid containing the DHMBA, and after 24 and 48 hours of the culture, the numbers of cells adhering to the bottom surface of the dish were counted to examine the action of cell death induction (Fig. 5). The cell deaths of the PC-3 (Figs. 5A and 5B) and DU-145 (Figs. 5D and 5E) were significantly increased when the cells were cultured in the presence of the DHMBA (1, 10, 100, or 1000 µM). These increases were not caused in the presence of the caspase-3 inhibitor (10 µM) (Figs. 5C and SF).

The culture with the DHMBA (10 µM) increased the levels of caspase 3 and activated caspase 3 (cleaved caspase-3) (Fig. 5G). These results suggested that the DHMBA induced apoptosis-like cell death.

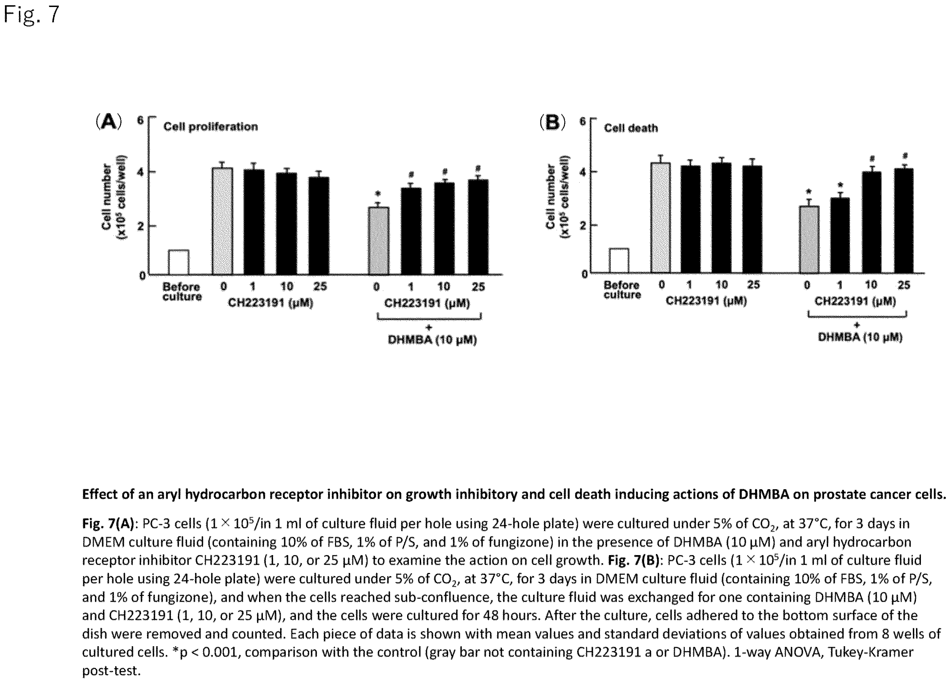

The gemcitabine has been used clinically as a cancer therapeutic agent to induce many cancer cell deaths. This action is based on an action to inhibit DNA synthesis of cell nuclei. Culturing the PC-3 cells in the presence of gemcitabine (1, 10, 50, 100, and 250 nM) for three days caused significant cell deaths (Fig. 7A). The cell death inducing action on PC-3 of the DHMBA (1 and 10 µM) did not occur in the presence of gemcitabine (1 or 10 nM) (Fig. 7B).

Interestingly, the cell death inducing effect of the DHMBA (1 and 10 µM) was found to be significantly enhanced in the coexistence of gemcitabine (100 nM) (Fig. 7B). These results suggested that the DHMBA was partly associated with the process of the cell death inducing action of gemcitabine.

### (Cell Growth Inhibitory Effect and Cell Death Inducing Effect of DHMBA Are Mediated by Aryl Hydrocarbon Receptor)

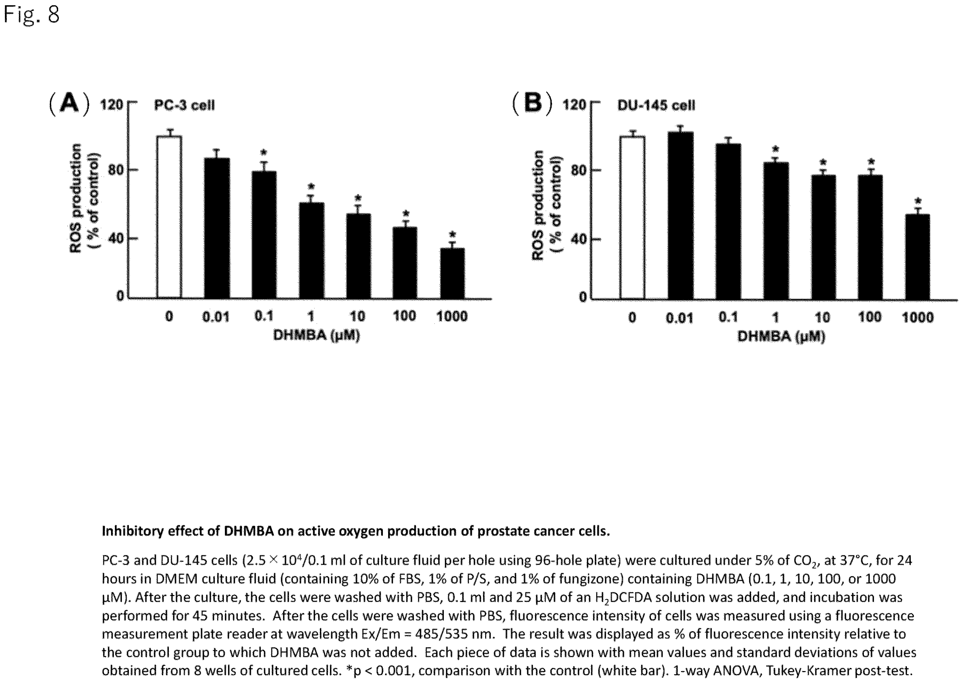

The aryl hydrocarbon receptor (AHR) binds to nuclear translocation protein factors in a cytoplasm and binds to genes by nuclear translocation, bringing drug-metabolizing enzymes and many other gene expressions. The DHMBA was inferred to bind to the AHR and produce the gene expression action. To demonstrate this, CH223191, the specific inhibitor of AHR, was used to examine whether the cell growth action and cell death inducing action of the DHMBA (10 µM) occurred (Fig. 8).

In the presence of CH223191 (1, 10, or 25 µM), the PC-3 cells were cultured for three days to examine the action on cell growth, and no significant effect was expressed (Fig. 8A). Similarly, the cells cultured for three days did not cause the cell death induction in sub-confluent state even when the CH223191 (1, 10, or 25 µM) was added and the cells were cultured for 48 hours (Fig. 8B). These results showed that the CH223191 at the concentration used in the experiment did not result in any detrimental effects on the cells. Thus, in the presence of this CH223191 (1, 10, or 25 µM), the cell growth inhibitory effect or the cell death inducing effect of the DHMBA (10 µM) was found not to be expressed. From the knowledge, it was inferred that a part of the action of the DHMBA was associated with the gene expression through the AHR.

### (Inhibitory Effect of DHMBA on Active Oxygen Production of Prostate Cancer Cells)

The DHMBA is well known to be an antioxidant. Therefore, the effect on the production of the active oxygen of the prostate cancer cells PC-3 and DU-145 was examined (Fig. 8).

In the presence of the DHMBA (1, 10, 100, and 1000 µM), significant inhibition was recognized when the amount of intracellular active oxygen production was measured after the cells were cultured for 24 hours. It was inferred from this result that a part of the effect of the DHMBA on prostatic cells was due to the decrease in active oxygen production.

### (DHMBA Inhibits Migration and Invasion Actions of Prostate Cancer Cells)

The PC-3 and DU-145 cells of human prostate cancer cells are metastatic cancer cells. The action on this metastatic nature was evaluated by examining the effects on the migration and invasion of the prostate cancer cells.

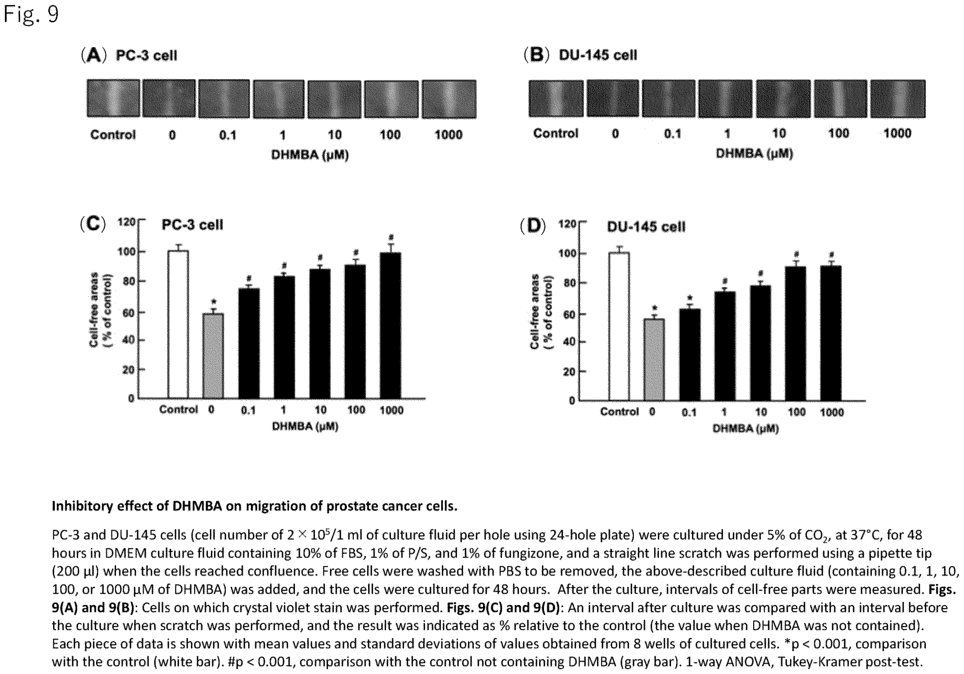

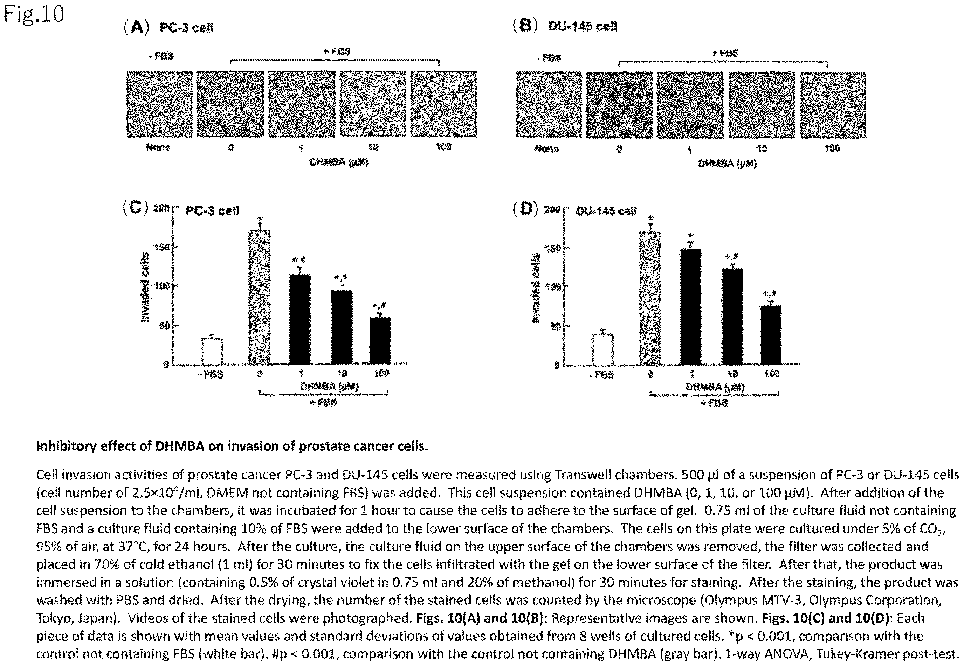

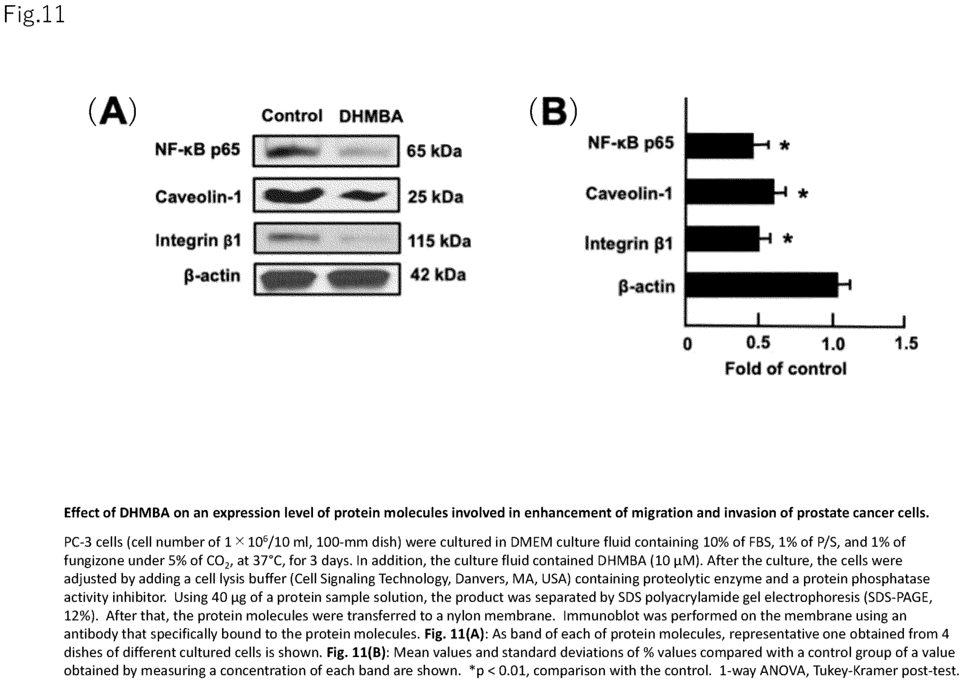

When the PC-3 cells (Figs. 9A and 9C) and the DU-145 cells (Figs. 9B and 9D) were cultured in the presence of the DHMBA (1, 10, 100, or 1000 µM), the migration was significantly inhibited. In addition, the invasion of the PC-3 cells (Figs. 10A and 10C) and the DU-145 cells (Figs. 10B and 10D) was also found to be significantly inhibited. Furthermore, it was found that when the expression levels of protein molecules involved in the enhancement of the migration and invasion of the prostate cancer cells were examined, the expression levels of NF-κB p65, caveolin-1, and integrin β1 were significantly decreased (Fig. 11).

### (Explanation of Each Drawing)

Fig. 1 is an explanatory diagram of performing an experiment on the inhibitory effect of 3,5-dihydroxy-4-methoxybenzyl alcohol (DHMBA) on the growth of the human prostate cancer PC-3 cells.

Fig. 1(A) is a drawing in which the PC-3 cells (the cell number of 1 × 10³/per well of a six-well plate) were cultured under 5% of CO₂, at 37°C, for nine days in a DMEM culture fluid (containing 10% of FBS, 1% of P/S, and 1% of fungizone) containing the DHMBA (1 or 10 µM).

Fig. 1(B) is a diagram of counting the number of colonies.

Fig. 1(C) is a diagram in which the PC-3 cells (1 × 10⁵ cells/per well of a 24-well plate) were cultured under 5% of CO₂, at 37°C, for one to six days in a DMEM culture fluid (containing 10% of FBS, 1% of P/S, and 1% of fungizone) containing the DHMBA (10 µM).

In Figs. 1(D) and 1(E), the PC-3 cells (1 × 10⁵ cells/per well of a 24-well plate) were cultured under 5% of CO₂, at 37°C, for three (Fig. 1D) and six (Fig. 1E) days in a DMEM culture fluid (containing 10% of FBS, 1% of P/S, and 1% of fungizone) containing the DHMBA (0.01, 0.1, 1, 10, 100, and 1000 µM). After the culture, the cells adhered to the bottom surface of the dish were removed and counted. Each piece of data is a diagram showing a mean value and a standard deviation of values obtained from eight wells of the cultured cells. **p* < 0.001, comparison with the control (white or gray bar). 1-way ANOVA, Tukey-Kramer post-test.

Fig. 2 is an explanatory diagram of performing an experiment on the inhibitory effect of 3,5-dihydroxy-4-methoxybenzyl alcohol (DHMBA) on the growth of human prostate cancer DU-145 cells.

Fig. 2(A) is a drawing in which the DU-145 cells (1 × 10³ cells/per well of a six-well plate) were cultured under 5% of CO₂, at 37°C, for nine days in a DMEM culture fluid (containing 10% of FBS, 1% of P/S, and 1% of fungizone) containing the DHMBA (1 or 10 µM).

Fig. 2(B) is a diagram of counting the number of colonies.

Fig. 2(C) is a diagram in which the DU-145 cells (1 × 10⁵ cells/per well of a 24-well plate) were cultured under 5% of CO₂, at 37°C, for one to six days in a DMEM culture fluid (containing 10% of FBS, 1% of P/S, and 1% of fungizone) containing the DHMBA (10 µM).

In Figs. 2(D) and 2(E), the DU-145 cells (1 × 10⁵ cells/per well of a 24-well plate) were cultured under 5% of CO₂, at 37°C, for three (Fig. 2D) and six (Fig. 2E) days in a DMEM culture fluid (containing 10% of FBS, 1% of P/S, and 1% of fungizone) containing the DHMBA (0.01, 0.1, 1, 10, 100, and 1000 µM). After the culture, the cells adhered to the dish were removed and counted. Each piece of data is a diagram showing a mean value and a standard deviation of values obtained from eight wells of the cultured cells. **p* < 0.001, comparison with the control (white or gray bar). 1-way ANOVA, Tukey-Kramer post-test.

Fig. 3 is an explanatory diagram of performing an experiment on the inhibitory effect of 3,5-dihydroxy-4-methoxybenzyl alcohol (DHMBA) on the growth of the human prostate cancer PC-3 cells in the presence of a cell growth inhibitor and an intracellular signaling inhibitor.

Figs. 3(A) and 3(B) are diagrams in which the PC-3 cells (1 × 10⁵ cells/ml/per well of a 24-well plate) were cultured under 5% of CO₂, at 37°C, for three days in a DMEM culture fluid (containing 10% of FBS, 1% of P/S, and 1% of fungizone) containing the DHMBA (10 µM) containing cell cycle inhibitors, roscovitine (10 and 100 nM), butyrate (10 and 100 µM), and sulforaphane (1 and 10 nM).

Figs. 3(C) and 3(D) are diagrams in which the PC-3 cells (1 × 10⁵ cells/ml/per well of a 24-well plate) were cultured under 5% of CO₂, at 37°C, for three days in a DMEM culture fluid (containing 10% of FBS, 1% of P/S, and 1% of fungizone) containing DHMBA (10 µM) containing wortmannin (10 or 100 nM), PD98059 (1 or 10 µM), and staurosporine (1 or 10 nM). After the culture, the cells adhered to the dish were removed and counted. Each piece of data is shown with a mean value and a standard deviation of values obtained from eight wells of the different cultured cells. **p* < 0.001, comparison with the control (gray bar). 1-way ANOVA, Tukey-Kramer post-test.

Fig. 4 is an explanatory diagram of performing an experiment on the effect of the DHMBA on the expression level of protein molecules involved in the enhancement of cell growth of the prostate cancer cells.

The PC-3 cells (1 × 10⁶ cells/10 ml, 100-mm dish) were cultured under 5% of CO₂, at 37°C, for three days in a DMEM culture fluid containing 10% of FBS, 1% of P/S, and 1% of fungizone. In addition, the culture fluid contained the DHMBA (10 µM). After the culture, the cells were adjusted by adding a cell lysis buffer (Cell Signaling Technology, Danvers, MA, USA) containing proteolytic enzyme and a protein phosphatase activity inhibitor. Using 40 µg of a protein sample solution, the product was separated by SDS polyacrylamide gel electrophoresis (SDS-PAGE, 12%). After that, the protein molecules were transferred to a nylon membrane. Immunoblot was performed on the membrane using an antibody that specifically bound to the protein molecules.

The band of each of the protein molecules is a drawing showing the representative one obtained from four dishes of the different cultured cells.

The drawing shows a mean value and a standard deviation of % values compared with a control group of a value obtained by measuring a concentration of each band. **p* < 0.01, comparison with the control. 1-way ANOVA, Tukey-Kramer post-test.

Fig. 5 is an explanatory diagram of performing an experiment on the effect of 3,5-dihydroxy-4-methoxybenzyl alcohol (DHMBA) on the cell death of the human prostate cancer PC-3 and DU-145.

In the drawing, the PC-3 (Figs. 5(A) and 5(B)) and DU-145 (Figs. 5(D) and 5(E)) cells (1 × 10⁵/in 1 ml of a culture fluid per hole using a 24-hole plate) were cultured under 5% of CO₂, at 37°C, for three days in a DMEM culture fluid (containing 10% of FBS, 1% of P/S, and 1% of fungizone), and when the cells reached sub-confluence, the culture fluid was exchanged for the one containing the DHMBA (0.01, 0.1, 1, 10, 100, or 1000 µM), and the cells were cultured for 24 and 48 hours. In the drawing, after the culture, the cells adhering to the dish were counted.

In another experiment, after the culture for three days, when the cells reached sub-confluence, for the PC-3 (Fig. 5(C)) or DU-145 (Fig. 5(F)) cells, the culture fluid was exchanged for a culture fluid containing the DHMBA (1 or 10 µM) and a caspase-3 inhibitor (10 µM), and the cells were counted after the culture for 48 hours. Further, after the culture for three days, when the cells reached sub-confluence, the culture fluid was exchanged for a culture fluid obtained by adding gemcitabine (0.1, 1, 10, 50 or 100 nM) or CH223191 (1, 10, and 25 µM) to a DMEM culture fluid containing the DHMBA (1 or 10 µM) or not containing the DHMBA, and the cells were counted after the culture for 48 hours. After the culture, the cells adhered to the dish were removed and counted. Each piece of data is shown with a mean value and a standard deviation of values obtained from eight wells of the different cultured cells. **p* < 0.001, comparison with the control (gray bar). 1-way ANOVA, Tukey-Kramer post-test.

In Fig. 5(G), the PC-3 cells (1 × 10⁶ cells/10 ml, 100-mm dish) were cultured under 5% of CO₂, at 37°C, for three days in a DMEM culture fluid containing 10% of FBS, 1% of P/S, and 1% of fungizone. In addition, the culture fluid contained the DHMBA (10 µM). After the culture, the cells were adjusted by adding a cell lysis buffer (Cell Signaling Technology, Danvers, MA, USA) containing proteolytic enzyme and a protein phosphatase activity inhibitor. Using 40 µg of a protein sample solution, the product was separated by SDS polyacrylamide gel electrophoresis (SDS-PAGE, 12%). After that, the protein molecules were transferred to a nylon membrane. Immunoblot was performed on the membrane using an antibody that specifically bound to the protein molecules.

The band of each of the protein molecules is a drawing showing the representative one obtained from four dishes of the different cultured cells.

The drawing shows a mean value and a standard deviation of % values compared with the control group of a value obtained by measuring a concentration of each band. **p* < 0.01, comparison with the control. 1-way ANOVA, Tukey-Kramer post-test.

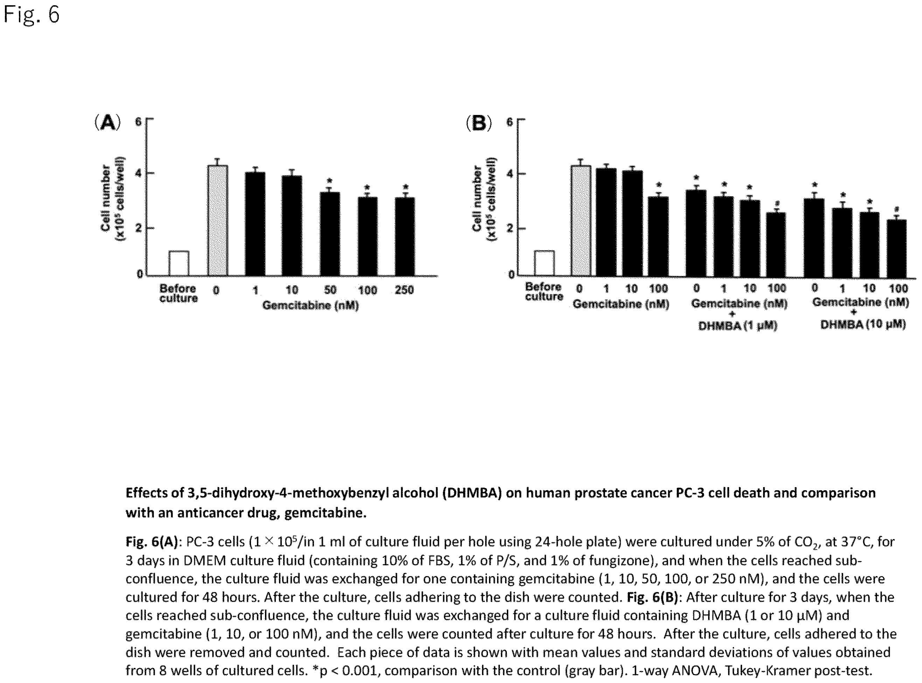

Fig. 6 is an explanatory diagram of an experiment on the effect of 3,5-dihydroxy-4-methoxybenzyl alcohol (DHMBA) on human prostate cancer PC-3 cell death and the comparison with an anticancer drug, gemcitabine.

The PC-3 cells (1 × 10⁵/in 1 ml of a culture fluid per hole using a 24-hole plate) were cultured under 5% of CO₂, at 37°C, for three days in a DMEM culture fluid (containing 10% of FBS, 1% of P/S, and 1% of fungizone), and when the cells reached sub-confluence, the culture fluid was exchanged for the one containing the gemcitabine (1, 10, 50, 100, or 250 nM), and the cells were cultured for 48 hours. In the drawing, after the culture, the cells adhering to the dish were counted.

After the culture for three days, when the cells reached sub-confluence, the culture fluid was exchanged for a culture fluid containing the DHMBA (1 or 10 µM) and gemcitabine (1, 10, or 100 nM), and the cells were counted after the culture for 48 hours. After the culture, the cells adhered to the dish were removed and counted. Each piece of data is diagramed to show a mean value and a standard deviation of values obtained from eight wells of the cultured cells. **p* < 0.001, comparison with the control (gray bar). 1-way ANOVA, Tukey-Kramer post-test.

Fig. 7 is an explanatory diagram of performing an experiment on the effect of the aryl hydrocarbon receptor inhibitor on the growth inhibitory and cell death inducing actions of the DHMBA on the prostate cancer cells.

In the drawing, the PC-3 cells (1 × 10⁵/in 1 ml of a culture fluid per hole using a 24-hole plate) were cultured under 5% of CO₂, at 37°C, for three days in a DMEM culture fluid (containing 10% of FBS, 1% of P/S, and 1% of fungizone) in the presence of the DHMBA (10 µM) and an aryl hydrocarbon receptor inhibitor CH223191 (1, 10, or 25 µM) to examine the action on cell growth.

The PC-3 cells (1 × 10⁵/in 1 ml of a culture fluid per hole using a 24-hole plate) were cultured under 5% of CO₂, at 37°C, for three days in a DMEM culture fluid (containing 10% of FBS, 1% of P/S, and 1% of fungizone), and when the cells reached sub-confluence, the culture fluid was exchanged for the one containing the DHMBA (10 µM) and the CH223191 (1, 10, or 25 µM), and the cells were cultured for 48 hours. After the culture, the cells adhered to the bottom surface of the dish were removed and counted. Each piece of data is diagramed to show a mean value and a standard deviation of values obtained from eight wells of the cultured cells. **p* < 0.001, comparison with the control (a gray bar not containing CH223191 a or DHMBA). 1-way ANOVA, Tukey-Kramer post-test.

Fig. 8 is an explanatory diagram of performing an experiment on the inhibitory effect of the DHMBA on the active oxygen production of the prostate cancer cells.

The PC-3 and DU-145 cells (2.5 × 10⁴/0.1 ml of a culture fluid per hole using a 96-hole plate) were cultured under 5% of CO₂, at 37°C, for 24 hours in a DMEM culture fluid (containing 10% of FBS, 1% of P/S, and 1% of fungizone) containing the DHMBA (0.1, 1, 10, 100, or 1000 µM). After the culture, the cells were washed with PBS, 0.1 ml and 25 µM of an H₂DCFDA solution was added, and incubation was performed for 45 minutes. After the cells were washed with PBS, a fluorescence intensity of the cells was measured using a fluorescence measurement plate reader at a wavelength Ex/Em = 485/535 nm. The result was displayed as % of fluorescence intensity relative to the control group to which the DHMBA was not added. Each piece of data is a diagram showing a mean value and a standard deviation of values obtained from eight wells of the cultured cells. **p* < 0.001, comparison with the control (white bar). 1-way ANOVA, Tukey-Kramer post-test.

Fig. 9 is an explanatory diagram of performing an experiment on the inhibitory effect of the DHMBA on the migration of the prostate cancer cells.

The PC-3 and DU-145 cells (the cell number of 2 × 10⁵/1 ml of a culture fluid per hole using a 24-hole plate) were cultured under 5% of CO₂, at 37°C, for 48 hours in a DMEM culture fluid containing 10% of FBS, 1% of P/S, and 1% of fungizone, and a straight line scratch was performed using a pipette tip (200 µl) when the cells reached confluence. Free cells were washed with PBS to be removed, the above-described culture fluid (containing 0.1, 1, 10, 100, or 1000 µM of the DHMBA) was added, and the cells were cultured for 48 hours. After the culture, intervals of cell-free parts were measured.

Figs. 9(A) and 9(B) are drawings of the cells on which crystal violet stain was performed.

In Figs. 9(C) and 9(D), an interval after culture was compared with an interval before the culture when scratch was performed, and the result was indicated as % relative to the control (the value when the DHMBA was not contained). Each piece of data is a diagram showing a mean value and a standard deviation of values obtained from eight wells of the cultured cells. **p* < 0.001, comparison with the control (white bar). #p < 0.001, comparison with the control not containing the DHMBA (gray bar). 1-way ANOVA, Tukey-Kramer post-test.

Fig. 10 is an explanatory diagram of performing an experiment on the inhibitory effect of the DHMBA on the invasion of the prostate cancer cells.

The cell invasion activities of the prostate cancer PC-3 and DU-145 cells were measured using the Transwell chambers. 500 µl of a suspension of the PC-3 or DU-145 cells (the cell number of 2.5 × 10⁴/ml, the DMEM not containing FBS) was added. This cell suspension contained the DHMBA (0, 1, 10, or 100 µM). After the addition of the cell suspension to the chambers, it was incubated for one hour to cause the cells to adhere to the surface of the gel. 0.75 ml of the culture fluid not containing the FBS and a culture fluid containing 10% of FBS were added to the lower surface of the chambers. The cells on the plate were cultured under 5% of CO₂, 95% of air, at 37°C, for 24 hours. After the culture, the culture fluid on the upper surface of the chambers was removed, the filter was collected and placed in 70% of cold ethanol (1 ml) for 30 minutes to fix the cells infiltrated with the gel on the lower surface of the filter. After that, the product was immersed in a solution (containing 0.5% of crystal violet in 0.75 ml and 20% of methanol) for 30 minutes for staining. After the staining, the product was washed with PBS and dried. After the drying, the number of the stained cells was counted by the microscope (Olympus MTV-3, Olympus Corporation, Tokyo, Japan). Videos of the stained cells were photographed.

Figs. 10(A) and 10(B) are diagrams showing the representative images.

In Figs. 10(C) and 10(D), each piece of data is a diagram showing a mean value and a standard deviation of values obtained from eight wells of the cultured cells. **p* < 0.001, comparison with the control not containing the FBS (white bar). #p < 0.001, comparison with the control not containing the DHMBA (gray bar). 1-way ANOVA, Tukey-Kramer post-test.

Fig. 11 is an explanatory diagram of performing an experiment on the effect of the DHMBA on the expression level of protein molecules involved in the enhancement of migration and invasion of the prostate cancer cells.

The PC-3 cells (the cell number of 1 × 10⁶/10 ml, 100-mm dish) were cultured in a DMEM culture fluid containing 10% of FBS, 1% of P/S, and 1% of fungizone under 5% of CO₂, at 37°C, for three days. In addition, the culture fluid contained the DHMBA (10 µM). After the culture, the cells were adjusted by adding a cell lysis buffer (Cell Signaling Technology, Danvers, MA, USA) containing proteolytic enzyme and a protein phosphatase activity inhibitor. Using 40 µg of a protein sample solution, the product was separated by SDS polyacrylamide gel electrophoresis (SDS-PAGE, 12%). After that, the protein molecules were transferred to a nylon membrane. Immunoblot was performed on the membrane using an antibody that specifically bound to the protein molecules.

The band of each of the protein molecules is a drawing showing the representative one obtained from four dishes of the different cultured cells.

The drawing shows a mean value and a standard deviation of % values compared with a control group of a value obtained by measuring a concentration of each band. **p* < 0.01, comparison with the control. 1-way ANOVA, Tukey-Kramer post-test.

### (Consideration)

Prostate cancer is a metastatic cancer that ranks the second in the cancer-related causes of death of males. Therefore, further development of more effective treatments for metastatic prostate cancer is required.

The DHMBA is a phenolic antioxidant isolated and identified from marine oysters originally.

In the present invention, experiments were conducted to find whether the DHMBA expressed the inhibitory effect on the metastatic human prostate cancer cells PC-3 and DU-145 cells.

Consequently, the DHMBA was found to inhibit cell migration and invasion associated with colony formation of cancer cells, cell growth, active oxygen production, and metastasis of cells, and additionally to exert the action to induce cancer cell death. Thus, the present invention provides a new method in the treatment of metastatic prostate cancer.

The growth inhibitory effect of the DHMBA on the prostate cancer cells was not expressed when the cells were cultured in the presence of the cell cycle inhibitors, roscovitine, butyrate, or sulforaphane. Butyrate inhibits the accumulation of cdc2 mRNA at the G1 anaphase of the cell cycle and disturbs the enhancement at the prophase and the anaphase of the G1 phase. Roscovitine is a selective, strong inhibitor of cyclin-dependent kinases cdc2, cdk2 and cdk5. Sulforaphane brings inhibition of cell division cycle at the G2/M phase associated with phosphorylation in the cell cycle mediated by checkpoint kinase.

Thus, it is considered that the DHMBA disturbs the cell growth cycle at the G1 and G2 M phases. Furthermore, the growth inhibitory effect of the DHMBA on the PC-3 cells was not recognized in the presence of wortmannin of the PI3K inhibitor, PD98059 of the MAPK inhibitor, or staurosporine of the protein kinase C inhibitor causing the PC-3 cell growth inhibition.

The cell growth inhibitory effect of the DHMBA was inferred to be caused by also acting on the intracellular signaling process that promotes the cell growth. Therefore, when the expression levels of Ras, Akt, MAPK, phospho-MAPK, and mTOR, which were involved in cell growth, were examined, these factors were found to be decreased. Furthermore, it was revealed that the expression levels of p53, p21, Rb, and regucalcin, which were tumor suppressor genes involved in cell growth inhibition, were increased.

Thus, the cancer cell growth inhibitory effect of the DHMBA was inferred to be exerted by acting on a wide range of cell signaling process.

In addition, the DHMBA was revealed to induce the death of the prostate cancer PC-3 and DU-145 cells. This knowledge inferred that the cell death inducing effect partially contributed to exerting the cell growth inhibitory effect of the DHMBA. The cell death inducing action of the DHMBA was inferred to be associated with the decrease in the expression levels of caspase-3, which was involved in the degradation of cell nuclear DNA. Interestingly, in the presence of gemcitabine, which is used clinically to treat cancer, a significant cell death inducing effect was increased when the DHMBA was used in combination. From the knowledge, the combination of the DHMBA of medical food factor and the gemcitabine, anticancer drug, was considered to be helpful for effective cancer treatment.

The cell growth inhibitory effect or the cell death inducing action of the DHMBA was not expressed in the coexistence of the specific inhibitor CH223191 of the aryl hydrocarbon receptor (AHR). The AHR binds to AHR binding nuclear translocation protein, performs nuclear translocation to the nucleus and binds to DNA, leading to the induction of a wide variety of genetic proteins. Among them, enzymatic protein involved in drug metabolism is present. The AHR was originally discovered from the study of strong binding to polychlorinated hydrocarbon, 2,3,7,8-tetrachlorodibenzo-p-dioxin (TCDD). The TCDD is known to exert the growth inhibitory effect on colon cancer and liver cancer cells. It also binds to in vivo compounds, nutritional isoflavones, and the like. The growth inhibitory action and the cell death inducing effect of the DHMBA on the prostate cancer cells were inferred to be partially exerted through binding to the AHR and by acting on the gene-expression.

The DHMBA is known as an antioxidant. The DHMBA was found to inhibit the active oxygen production of the PC-3 and DU-145 cells as the prostate cancer cells. It was inferred from the knowledge that the DHMBA partially contributed to the growth inhibition of prostate cancer cells and to the expression of the cell death inducing action.

Furthermore, the DHMBA was found to inhibit the migration and invasion of the metastatic prostate cancer cells, PC-3 and DU-145 cells. The DHMBA decreased the expression levels of NF-κB p65, caveolin-1, and integrin β1 of molecules involved in the metastasis of cancer cells. It was considered from the knowledge that the DHMBA inhibited the metastatic activity of prostate cancer cells.

### (Conclusion)

In the present invention, novel knowledge has been elucidated that the DHMBA, a new marine compound, exerts actions, such as inhibiting the growth of metastatic human prostate cancer cells, inducing the cell death, and inhibiting the metastatic activity of cancer cells.

3,5-dihydroxy-4-methoxybenzyl alcohol (DHMBA) is a medical food factor and has minimal bio-virulence. A growth inhibitor of metastatic human prostate cancer cells having a growth inhibitory action on metastatic human prostate cancer cells with an active ingredient of the DHMBA, a cell death inducer of metastatic human prostate cancer cells having a cell death inducing action on metastatic human prostate cancer cells with an active ingredient of 3,5-dihydroxy-4-methoxybenzyl alcohol (DHMBA), and an inhibitor of cell migration and invasion actions of metastatic human prostate cancer cells having inhibitory actions on migration and invasion actions of metastatic human prostate cancer cells with an active ingredient of 3,5-dihydroxy-4-methoxybenzyl alcohol (DHMBA) can be claimed to be valuable as a novel therapeutic agent for metastatic prostate cancer cells.

## Claims

1. A growth inhibitor of metastatic human prostate cancer cells having a growth inhibitory action on metastatic human prostate cancer cells with an active ingredient of 3,5-dihydroxy-4-methoxybenzyl alcohol (DHMBA).

2. A cell death inducer of metastatic human prostate cancer cells having a cell death inducing action on metastatic human prostate cancer cells with an active ingredient of 3,5-dihydroxy-4-methoxybenzyl alcohol (DHMBA).

3. An inhibitor for cell migration and invasion actions of metastatic human prostate cancer cells having inhibitory actions on migration and invasion of metastatic human prostate cancer cells with an active ingredient of 3,5-dihydroxy-4-methoxybenzyl alcohol (DHMBA).
